# EUROPEAN PATENT SPECIFICATION

(11) **EP 1 261 283 B1**
(45) Date of publication and mention of the grant of the patent: **04.05.2005**
(21) Application number: 01914255.3
(22) Date of filing: 08.03.2001
(51) Int. Cl.: A61B 17/34

(54) **APPARATUS FOR HAIR TRANSPLANTATION**
GERÄT ZUR HAARIMPLANTATION
APPAREIL D'IMPLANTATION DE CHEVEUX

(30) Priority: 08.03.2000 NO 20001182
(43) Date of publication of application: 04.12.2002
(73) Proprietor: Hair Line AS, 1807 Askim (NO)
(72) Inventor: Werner, Per Gunnar, 1454 Fagerstrand (NO)
(74) Representative: Johansson, Lars E.
(86) International application number: PCT/NO2001/000103
(87) International publication number: WO 2001/065996

(56) References cited:
- WO-A1-00/64379
- WO-A1-95/28896
- WO-A1-98/47434
- GB-A- 1 553 950
- US-A- 5 417 683
- US-A- 5 439 475

## Description

The invention relates to an apparatus for implantation of surgically excised hair roots, hereinafter called grafts, which have been surgically removed from, e.g., the area with abundant hair growth at the nape of the patient's neck to be implanted in areas with scant hair growth in the skin of this person's head.

After drill-out or excision the grafts are inserted in a container which is equipped with a large number of compartments, hereinafter called container compartments, for reception thereof. The filled container is then inserted in a lead-in channel in an implantation apparatus, by means of which it is carefully inserted into the skin of the head. The container is equipped with advancement devices, such as pawl devices, a toothed rack or the like which are engaged with corresponding advancement devices in the implantation apparatus. The container is advanced stepwise in the implantation apparatus and is positioned in such a manner that the grafts can be delivered to an implantation mechanism.

In addition to the reception device for the container, the implantation apparatus has a tubular body, hereinafter called a cannula, at the front of which is a cutting edge which can cut openings in the skin of the head, and whose tubular portion is suitable for guiding the grafts into the skin of the head, and furthermore the apparatus has an ejector pin which pushes the graft out of the container compartment through the cannula to the desired depth in the skin of the head.

Implantation apparatuses are previously known from, e.g., WO-A-9528896, which describes an implantation apparatus equipped with two cutting parts which perform an incision in the skin before extending the incision to form an opening in the skin for insertion of the graft. This opening is almost square since the knife edges which penetrate the skin are pulled outwards to each side for a distance approximately equal to the length of each knife. The skin is stretched to such an extent at the corners that it is unable to contract again when the knives are withdrawn. Moreover, the container used for storing the grafts according to this publication has separate, movable receptacles. These are moved into the skin, whereupon the graft is expelled while the receptacle is withdrawn. This requires a substantial expansion of the skin opening since not only the graft but also the receptacles have to be inserted in the skin opening.

This will most probably result in heavy bleeding, thus causing the grafts to be flushed out of the skin.

The above mentioned document has been used for the delineation of claim 1. In the two-past form. US-A-5 873 888 discloses a system for implantation of hair grafts. The system comprises movable containers for storage of grafts. The containers are pushed into a pistol-like instrument, fed forward step by step by the integrated advancement devices and delivered to the reception area. The publication describes a cannula-like insertion device which penetrates the skin of the head and guides the grafts in under the skin. According to this publication the cannula device has to be inserted and retracted manually. This results in the cannula and the ejector pin being withdrawn simultaneously. The cannula and the ejector pin will therefore pull out with them the graft which is still partly in contact with the point of the cannula, and the flow of blood will flush out the graft. The manner in which the pistol-like instrument is implemented allows no possibility of the ejector pin remaining inside the skin while the cannula is retracted, since they cannot be moved independently of each other.

The object of the invention is to remedy the said disadvantages of the known apparatuses.

This object is achieved by means of an apparatus for implanting hair grafts as specified in Claim 1.

In a preferred embodiment the control means will control the devices in such a manner that between steps b) and c) the cannula is moved to a first position and held still for a time interval which is either pre-programmed or is selected during use. The object of this is to make it easy for the user to pinpoint the location where the cannula has to be placed.

It will also be advantageous for the control means to control the devices in such a manner that between steps c) and d) the cannula is moved a predetermined distance backwards. The object of this is to make more room for the graft in the hole provided by the cannula.

In a specially preferred embodiment the transport devices for the cannula, the ejector and the container will be driven by servomotors or stepping motors, and the control means are composed of a programmed logic control or a microprocessor control. In this case the control means will preferably receive position and speed signals from signal transmitters which acknowledge that each movement of the devices has been performed and that the devices have reached their front and/or rear positions and/or positions therebetween.

The control means may further comprise programs for controlling interruptions in production, and/or rewinding of the container, and/or repair and testing of the apparatus.

The container used in the apparatus according to the invention will not move during the actual implantation but will be arranged in the correct position and then held still during implantation. The container is filled with one graft in each container compartment, and this graft is expelled by an ejector pin from the container compartment into the end of the cannula and on through it while the cannula is moved towards and into the skin. With its cutting edge the cannula will cut the skin without tearing it up. It will make a round opening which encloses the cannula due to the skin's elastic properties. Since the receptacle does not accompany the ejector pin, the round hole in the skin will be smaller than that obtained when using the apparatus according to NO-A-19981307. According to the invention the cannula will be retracted while the ejector pin is still located inside the skin, since it holds the graft during retraction of the cannula. The cannula's wall thickness is around 0.2 mm and thus the ejector pin is not substantially smaller diametrically than the round opening in the skin. The skin will therefore enclose the ejector pin as soon as the cannula is removed, thereby preventing blood from flowing out. When the ejector pin is subsequently removed, the skin will contract further and the graft will seal the opening quite effectively.

As regards the system known from US-A-5 873 888 it is not possible to move the cannula and the ejector pin independently of each other. The invention permits and is actually based on such an independent movement, since the cannula can be pushed all the way into the skin and then retracted slightly before the graft is inserted, thus making more room for the graft.

The apparatus according to the invention permits control of the movements of the container, the cannula and the ejector with regulation of speed, depth, withdrawal distance and initial position for the cannula and the ejector pin. The invention thereby provides an optimal implantation apparatus, with cutting of small, round holes in the skin, in addition to which the minimum number or no grafts are flushed out of the skin.

The apparatus according to the invention will now be explained in more detail with reference to drawings, in which:
figure 1a is a longitudinal section through the implantation apparatus,
figure 1b is a cross section of the implantation apparatus,
figure 2 is an enlarged view of the front end of the apparatus,
figure 3a is another enlarged view of the front end of the apparatus,
figure 3b illustrates a detail of the cannula,
figure 4 is a cross section of the apparatus at the advancement mechanism for the container,
figure 5 is a horizontal section on a level with the cannula of the front part of the apparatus,
figure 6 illustrates the use of the apparatus.

As mentioned earlier, the apparatus is preferably equipped with three servomotors or stepping motors, each with a suitable gear. The servomotors drive the transport devices, and they are controlled by freely programmable electronic control means. Control may be undertaken by each motor being controlled separately or by having a central control unit for the three motors. The control means will be capable of starting and stopping each of the movements at any point between the extreme points, and the control means will be capable of controlling the speed of motion during the entire implantation process. In addition to the main program, the control means can store a number of subprograms which may be useful in connection with interruptions in production, cleaning, rewinding of container tapes, repair and testing etc.

The apparatus may be designed to be held by a pistol grip with a built-in switch. The apparatus may also act well without the pistol grip and with a photo-operated switch.

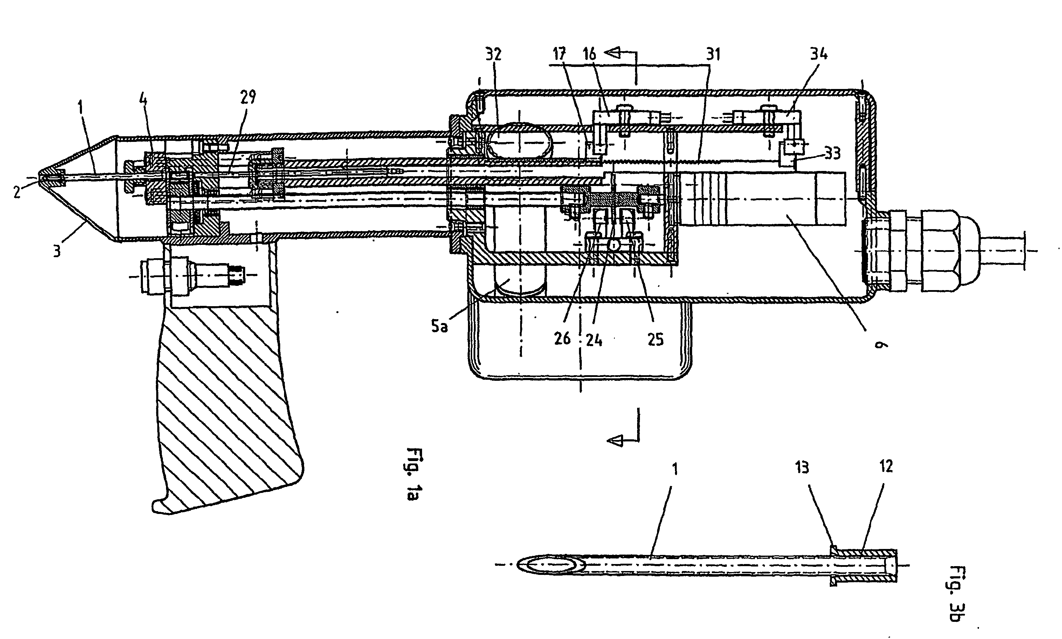

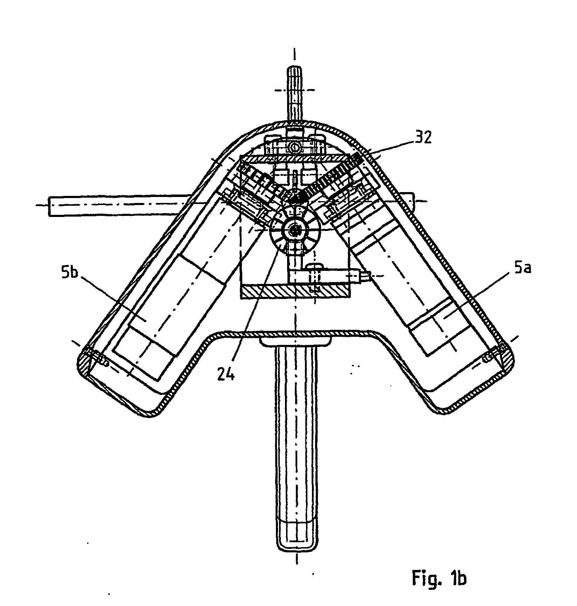

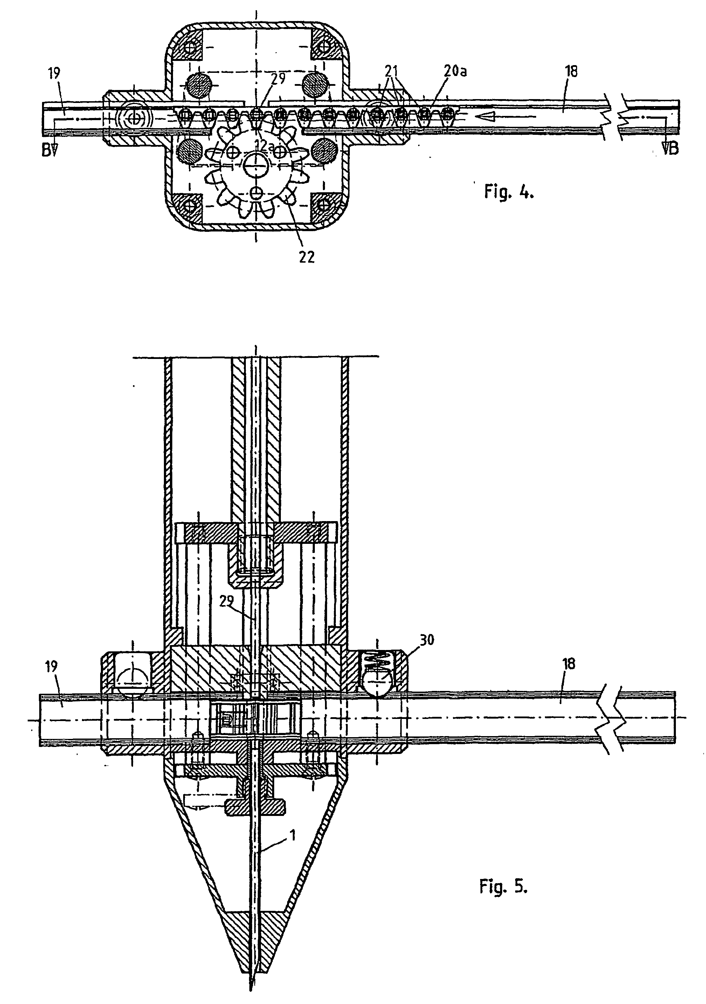

Fig. 1a is a longitudinal section through an implantation apparatus according to the invention, comprising fixing and transport devices which are driven by motors and which can bring the container for the grafts, the cannula and the ejector for the graft to the desired positions in the desired order and at the desired speeds. The figure illustrates a cannula 1, with a cutting edge 2, a nozzle 3, a clamping plate 4 for the cannula (which will be described in more detail later) and two of the motors, the motor 5a for movement of the ejector 29 and the motor 6 for movement of the container together with the signal transmitters, these parts being described in more detail later. In the preferred embodiment illustrated in the picture the apparatus's motors for guiding the cannula and the ejector are arranged slantingly relative to each other (figs. 1b, 5a and 5b) and in such a manner that the user can lay the pistol along the forearm so that the cleft between the "motor housings" is firmly supported against the forearm/wrist.

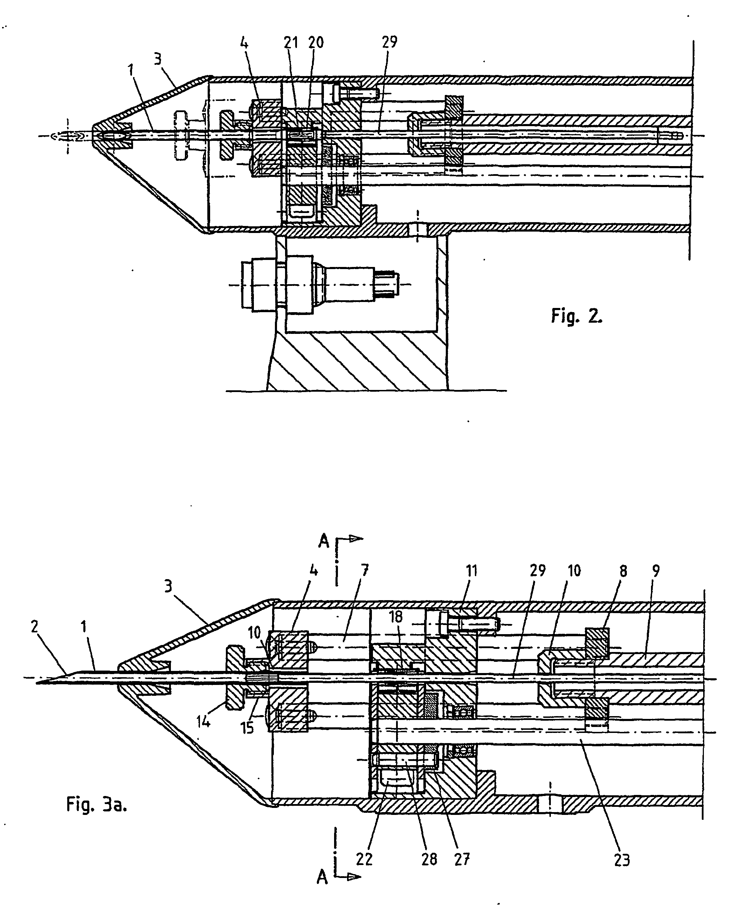

Fig. 2 is an enlarged section of the front part of the apparatus. The apparatus comprises a cannula 1, which in the figure is illustrated in its fully withdrawn position. The cannula's 1 cutting edge 2 is located approximately 1 mm inside the opening in the apparatus's nozzle 3. For the sake of clarity the term front part or forward direction will hereinafter be employed in connection with the part of the apparatus located closest to the nozzle 3, and rear part or backward direction in connection with the opposite part of the apparatus. The cannula 1 is attached to a clamping plate 4 which can be moved to any position within a front and a rear limit in the apparatus's longitudinal direction, as indicated by dot-and-dash lines. This will be explained in more detail later.

Fig. 3a is another enlarged section of the front part of the apparatus, where the cannula 1 is rotated 90°, thus illustrating more clearly the design of the cannula's 1 front cutting part 2, and where the clamping plate 4 and the cannula 1 are in the programmed position which is located further forwards. The figure further illustrates that the clamping plate 4 is attached to the front end of a number of pillars 7 which are attached at the opposite, rear end to a holding plate 8 which in turn is mounted on to a tubular rack 9 and is secured thereto by a barrel nut 10. The pillars 7 are guided by a number of precise borings through a flange 11.

The clamping plate 4 has an precise boring for positioning and securing the cannula 1 and a bushing 12 with a flange 13 (see fig. 3b) welded or glued to the cannula 1. A hollow screw 14 which is passed over the cannula 1, is screwed into the internally threaded neck 15 of the clamping plate 4, pressing the flange 13 with the cannula 1 securely against the clamping plate 4. The hollow screw 14 should be designed to be able to be screwed and unscrewed by means of the fingers, and this can be achieved by making the head of the screw a suitable size and having it serrated. The insertion and removal of the cannula 1 are only undertaken after removal of the nozzle 3.

The cannula 1 is driven by a motor to whose shaft is mounted a pinion or pinion (not shown) which drives the tubular rack 9 from a rear position illustrated in figures 2 and 5 to a front position illustrated in figure 3a. The rack 9 is in the form of a thick-walled tube, where the task of the tube wall is to receive the rack teeth which are directly incorporated in it. The rack 9 is designed to be able to move slightly further than the distance necessary for the cannula 1 to penetrate the skin deeply enough, i. e. preferably more than 8-9 mm. The rack's 9 position is identified by a fixed photocell 16 and a flag 17 mounted at a suitable position on the rack 9 (fig. 1a) and which allows light to pass through to the photocell 16 when the rack is near one of the extreme positions. The control means is so programmed that the rack moves the required distance from the signal point in both directions.

The apparatus further comprises receiving devices in the form of a lead-in and an exit channel 18 and 19 respectively for a container 20 with hair grafts 21 (see figs. 3a and 4). In order to effect movement of the container, the apparatus includes a transport device in the form of a toothed ratchet wheel 22. A motor is provided for driving the ratchet wheel 22 which is engaged with the container's 20 rack teeth in such a manner that the container 20 can be advanced with great accuracy to the next withdrawal position and secured in this position while the ejector 29 expels the graft 21 from the container compartment on to the delivery location in the receiver skin and the ejector 29 is withdrawn to its starting position. The ratchet wheel 22 is releasably attached to a shaft 23, which is driven by the motor 6 (figure 1). Mounted in connection with the shaft 23 is a signal disc 24 with radially arranged very narrow grooves with the same angular spacing as the ratchet wheel's 22 toothed angular spacing. In figure 4 there is illustrated a ratchet wheel or spur pinion 22 which in the present example has 12 teeth. This means that the angular spacing between the teeth is 30°, and the angular spacing of the gaps in the signal disc 24 is the same, viz. 30°. On one side of the signal disc 24 is a small light source 25 and on the other side a photocell 26. When the spur pinion 22 is accurately located in a delivery position, a beam of light will pass from the light source 25 via a gap in the signal disc 24 to the photocell 26. The photocell will transmit a signal to the control means which stops the spur pinion's 22 movement. The shaft has a driving device, e.g. a welded-on disc 27 with driving pins 28 which are engaged with borings in the ratchet wheel 22.

Fig. 4 is a section (A-A in fig. 3a), where the relative positions of the ratchet wheel 22, the container 20 and the ejector 29 are illustrated. The arrow illustrates the direction of feed for the container 20, which is located in the lead-in channel 18 and is on its way into the exit channel 19. The ratchet wheel 22 in the drawing is fed stepwise 30° for each graft as mentioned above. Other intervals than 30° may be chosen. The container compartments, where the grafts 21 are located, are preferably mounted centrally in the rack-shaped container's 20 teeth 20a. These teeth fit exactly into the ratchet wheel's 22 toothed openings 22a. In the middle of the container compartment the ejector 29 advances and passes through it, thus expelling the graft 20 from the container compartment into the cannula 1.

Fig. 5 is a horizontal section (B-B) on a level with the centre line of the ejector 29 and the cannula 1. The figure illustrates how the lead-in and exit channels 18, 19 are kept in place by two spherical snap fasteners 30 which securely grip countersinks in the two channels 18 and 19. (The figure does not show the actual container).

As regards the ejector 29, this is movably attached to a rack 31 which is engaged with a pinion 32 which is driven by a motor 5. At its innermost end the ejector 29 has a thread which is screwed into a threaded hole at the end of the rack 31, with the result that the ejector 29 follows the movements of the rack 31 backwards and forwards. The rack 31 is moved in a precise guide track in the thick-walled, tubular rack 9. The rack 9 and the rack 31 can be moved backwards and forwards independently of each other. The rack 31 also has a flag 33 which refracts the beam of light to the photocell switch 34 when the rack 31 approaches the rear, withdrawn position. The resulting signal is processed by the control means, and the program ensures that the rack always moves the desired distance from this point.

The signal transmitters may be of the capacitive or inductive type, or they may be mechanical limit switches or photocell switches, as described in the example above.

It is expedient to start and stop the motors in a sufficiently smooth manner, for which purpose the control means has acceleration and retardation functions, so-called ramp functions. By means thereof the positioning accuracy is improved, since the motor's speed is greatly reduced just before it reaches the stopping point.

The invention's mode of operation will now be explained in more detail.

In broad outline it may be said that the invention provides a controllable and programmable interaction between the individual, independent movements of the container 18, the ejector 29 and the cannula 1 in order to push the graft 21 from the container 18 into the patient's scalp with as little stress as possible for the graft and the skin. With this object in view, the apparatus according to the invention has fixing and transport devices in addition to control means for the said devices.

The implantation process begins by a starting signal being given by the user by means of a switch. The ratchet wheel 22 is then rotated, thus causing the container 20 to assume a position where a filled container compartment is arranged in relation to the ejector 29 and the cannula 1. During this part of the process the cannula 1 is located in its rear position.

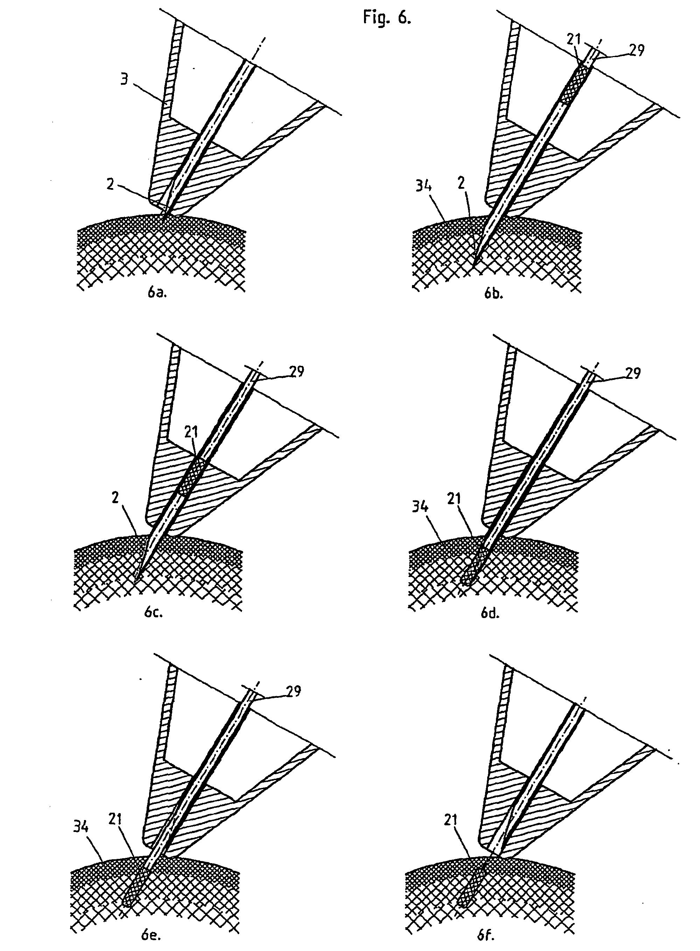

The ejector 29 is moved forwards in order to expel a graft 21 from the container 20 into the cannula 1 (figs. 2, 3a), and on into the cannula 1 to push the graft towards the cannula's 1 cutting edge 2 (figs. 6b and 6c).

In a preferred embodiment of the invention the cannula 1 is then moved slightly forwards to a position where it protrudes slightly from the nozzle 3 (fig. 6a). This may be done to make it easier for the user to place the cannula 1 in exactly the correct position before he presses the starting switch on the apparatus or before a predetermined time interval expires and the subsequent movements are activated.

The cannula 1 is then moved forwards simultaneously with the ejector 29. The ejector 29 and the cannula 1 will move forwards at approximately the same rate, - preferably in such a manner that the ejector 29 moves slightly faster than the cannula 1, in order to ensure that the movement of the graft 21 relative to the cannula 1 does not come to a stop. Since the static or rest friction between the graft 21 and the inside of the cannula 1 is greater than the dynamic or slide friction therebetween, it is an advantage that the graft 21 is moving all the time relative to the cannula 1.

The cannula's 1 movement will stop when the front position is reached, and it will either rest in this position, or in a preferred embodiment of the invention will be retracted slightly to rest in a position which provides more room for the graft 21. The graft 21 is now very close to the outlet (figure 6c).

The ejector 29 will continue its forward movement until it has pushed the graft 21 all the way down into the opening in the skin which the cannula 1 has cut. The ejector's 29 movement is then stopped. The graft 21 will thereby be located clamped between the inner wall of the cannula 1 on one side, the surrounding skin on the other side and the ejector 29 on the top. The dermis 34, which is very strong and elastic, will contract around the cannula 1 (figs. 6b to 6d).

The cannula 1 is then moved backwards until it reaches its rear position. The cannula 1 is thereby completely retracted into the nozzle 3, while the ejector 29 is still located in the programmed extreme position, with the result that it holds on to the graft 21 while the cannula 1 is withdrawn from the skin (fig. 6e).

After the cannula 1 has been moved a sufficient distance backwards to remove it from the skin, the ejector's 29 backward movement can begin. This movement will stop when it has reached the programmed, rear position. When the ejector 29 has been withdrawn from the skin, the dermis 34 will contract almost completely (fig. 6f). In most cases the skin will now hold on to the graft 21, and the opening will be so tightly sealed that only very small amounts of blood seep through.

After an interval, when the user places the cannula at a new point, he will press the start switch and the process will begin all over again.

## Claims

1. An apparatus for implanting hair grafts wherein the apparatus comprises an incision means (1), an ejector (29) which is arranged to act jointly with the incision means (1) and reception devices for receiving a container (20) for storing hair grafts (21), where the container (20) comprises a number of container compartments and each container (20) compartment contains a graft (21), and
where the apparatus comprises fixing and transport devices for the incision means (1), the ejector (29) and the container (20), where the devices can be moved independently of one another, the apparatus further comprising signal transmitters for registering and indicating the position of the incision means (1), the ejector (29) and the container (20), together with programmable control means for receiving signals from the signal transmitters as well as a starting signal from a user and for individual control of the devices,
**characterized in that** the incision means (1) is in the form of a cannula, and that the signal transmitters for registering and indicating the position of the cannula (1), the ejector (29) and the container (20), together with programmable control means for receiving signals from the signal transmitters as well as a starting signal from a user and for individual control of the devices, are enabled to:
a) move the container (20) to a withdrawal position where a filled container (20) compartment is arranged in relation to the ejector (29) and the cannula (1) and held still in this position,
b) move the ejector (29) forwards in order to expel a graft (21) from the container (20) compartment into the cannula (1),
c) move the ejector (29) and the cannula (1) forwards with a movement relative to each other in order to push the graft (21) towards the cannula (1)'s cutting edge (2),
d) stop the cannula (1) in a front position and hold it still while the ejector (29) continues to expel the graft (21),
e) stop the ejector (29) in a front position and hold it still while the cannula (1) is retracted,
f) move the ejector (29) and the cannula (1) backwards until their respective rear positions have been reached,
where the container (20) is secured in its' withdrawal position while the ejector (29) expels the graft (21) from the container (20) compartment and the ejector (29) is withdrawn to its starting position.

2. An apparatus according to claim 1,
**characterized in that** the control means control the devices in such a manner that between steps b) and c) the cannula (1) is moved to a first position and held still for an interval of time which is either pre-programmed or is selected during use.

3. An apparatus according to claim 1 or 2,
**characterized in that** the control means control the devices in such a manner that between steps c) and d) the cannula (1) is moved a predetermined distance backwards.

4. An apparatus according to one of the preceding claims,
**characterized in that** the transport devices for the cannula (1), the ejector (29) and the container (20) are driven by servomotors or stepping motors, and the control means are composed of a programmed logic control or a microprocessor control.

5. An apparatus according to one of the preceding claims,
**characterized in that** the control means receive position and speed signals from signal transmitters, which acknowledge that each movement of the devices has been performed and that the devices have reached their front and/or rear positions and/or positions therebetween.

6. An apparatus according to one of the preceding claims,
**characterized in that** the control means comprise programs for controlling interruptions in production, and/or rewinding of the container (20), and/or repair and testing of the apparatus.

## Patentansprüche

1. Vorrichtung zum Implantieren von Haartransplantaten, wobei die Vorrichtung eine Inzisionseinrichtung (1), eine Ausstoßeinrichtung (29), die so eingerichtet ist, dass sie eng mit der Inzisionseinrichtung (1) zusammenwirkt, und Aufnahmevorrichtungen zur Aufnahme eines Behälters (20) zum Lagern der Haartransplantate (21) umfasst, wobei der Behälter (20) eine Anzahl von Behälterfächern umfasst und jedes Fach eines Behälters (20) ein Haartransplantat (21) enthält, und wobei die Vorrichtung Feststell- und Transportvorrichtungen für die Inzisionseinrichtung (1), die Ausstoßeinrichtung (29) und den Behälter (20) umfasst, wobei die Feststell- und Transportvorrichtungen unabhängig voneinander bewegt werden können, wobei die Vorrichtung darüber hinaus Signalgeber zum Aufzeichnen und Anzeigen der Position der Inzisionseinrichtung (1), der Ausstoßeinrichtung (29) und des Behälters (20) zusammen mit programmierbaren Steuereinrichtungen umfasst, um Signale von den Signalgebern sowie ein Startsignal von einem Benutzer und zur individuellen Steuerung der Vorrichtungen zu empfangen,
**dadurch gekennzeichnet, dass**
die Inzisionseinrichtung (1) die Form einer Kanüle hat, und dass die Signalgeber zum Aufzeichnen und Anzeigen der Position der Kanüle (1), der Ausstoßeinrichtung (29) und des Behälters (20) zusammen mit den programmierbaren Steuereinrichtungen, um Signale von den Signalgebern sowie ein Startsignal von einem Benutzer und zur individuellen Steuerung der Vorrichtungen zu empfangen, aktiviert werden, um:
a) den Behälter (20) zu einer Entnahmeposition zu bewegen, an der ein befülltes Fach des Behälters (20) bezüglich der Ausstoßeinrichtung (29) und der Kanüle (1) angeordnet und in dieser Position still gehalten wird,
b) die Ausstoßeinrichtung (29) nach vorn zu bewegen, um ein Transplantat (21) aus dem Fach des Behälters (20) in die Kanüle (1) auszustoßen,
c) die Ausstoßeinrichtung (29) und die Kanüle (1) mit einer zueinander relativen Bewegung nach vorn zu bewegen, um das Transplantat (21) zur Schneide (2) der Kanüle (1) zu schieben,
d) die Kanüle (1) in einer vorderen Position anzuhalten und sie still zu halten, während die Ausstoßeinrichtung (29) fortfährt, das Transplantat (21) auszustoßen,
e) die Ausstoßeinrichtung (29) in einer vorderen Position anzuhalten und still zu halten, während die Kanüle (1) zurückgezogen wird,
f) die Ausstoßeinrichtung (29) und die Kanüle (1) nach hinten zu bewegen , bis ihre jeweiligen hinteren Positionen erreicht worden sind,
wobei der Behälter (20) in seiner Entnahmeposition festgesetzt ist, während die Ausstoßeinrichtung (29) das Transplantat (21) aus dem Fach des Behälters (20) holt, und die Ausstoßeinrichtung (29) in ihre Ausgangsposition zurückgefahren wird.

2. Vorrichtung nach Anspruch 1,
**dadurch gekennzeichnet, dass**
die Steuereinrichtungen die Vorrichtungen derart steuern, dass zwischen den Schritten b) und c) die Kanüle (1) zu einer ersten Position bewegt und eine Zeitdauer lang still gehalten wird, die entweder vorprogrammiert ist oder während des Gebrauchs ausgewählt wird.

3. Vorrichtung nach Anspruch 1 oder 2,
**dadurch gekennzeichnet, dass**
die Steuereinrichtungen die Vorrichtungen derart steuern, dass zwischen den Schritten c) und d) die Kanüle (1) eine vorbestimmte Strecke nach hinten bewegt wird.

4. Vorrichtung nach einem der vorhergehenden Ansprüche,
**dadurch gekennzeichnet, dass**
die Transportvorrichtung für die Kanüle (1), die Ausstoßeinrichtung (29) und der Behälter (20) durch Stell- oder Schrittmotoren angetrieben werden und die Steuereinrichtungen aus einer programmierten Logiksteuerung oder einer Mikroprozessorsteuerung bestehen.

5. Vorrichtung nach einem der vorhergehenden Ansprüche,
**dadurch gekennzeichnet, dass**
die Steuereinrichtungen Positions- und Geschwindigkeitssignale von den Signalgebern empfangen, welche bestätigen, dass jede Bewegung der Vorrichtungen durchgeführt worden ist und die Vorrichtungen ihre vorderen und/oder hinteren Positionen und/oder dazwischenliegende Positionen erreicht haben.

6. Vorrichtung nach einem der vorhergehenden Ansprüche,
**dadurch gekennzeichnet, dass**
die Steuereinrichtungen Programme umfassen, um Produktionsunterbrechungen und/oder ein Rücksetzen des Behälters (20) und/oder eine Reparatur und einen Prüflauf der Vorrichtung zu steuern.

## Revendications

1. Appareil pour implanter des greffes de cheveux, dans lequel l'appareil comprend un moyen d'incision (1), un éjecteur (29) qui est agencé pour fonctionner de façon conjointe avec le moyen d'incision (1), et des dispositifs de réception pour recevoir un récipient (20) pour stocker des greffes de cheveux (21), où le récipient (20) comprend un certain nombre de compartiments et chaque compartiment du récipient (20) contient une greffe (21) et où l'appareil comprend des dispositifs de fixation et de transport pour le moyen d'incision (1), l'éjecteur (29) et le récipient (20), où les dispositifs peuvent être déplacés indépendamment les uns des autres, l'appareil comprenant en outre des émetteurs de signaux pour enregistrer et indiquer la position du moyen d'incision (1), de l'éjecteur (29) et du récipient (20), associés à des moyens de commande programmables pour des signaux de réception provenant des émetteurs de signaux, ainsi qu'un signal de début provenant d'un utilisateur, et servant à la commande individuelle des dispositifs,
**caractérisé**
**en ce que** le moyen d'incision (1) se présente sous la forme d'une canule et en ce que les émetteurs de signaux pour enregistrer et indiquer la position de la canule (1), de l'éjecteur (29) et du récipient (20), associés à des moyens de commande programmables pour des signaux de réception provenant des émetteurs de signaux, ainsi qu'un signal de début provenant d'un utilisateur et servant à la commande individuelle des dispositifs, sont validés :
a) pour déplacer le récipient (20) jusqu'à une position de retrait où un compartiment rempli du récipient (20) est agencé en relation avec l'éjecteur (29) et la canule (1) et maintenu encore dans cette position,
b) pour faire avancer l'éjecteur (29) afin d'expulser une greffe (21) du compartiment du récipient (20), à l'intérieur de la canule (1),
c) à faire avancer l'éjecteur (29) et la canule (1) suivant un mouvement de l'un par rapport à l'autre, afin de pousser la greffe (21) vers le bord coupant (2) de la canule (1),
d) pour stopper la canule (1) dans une position frontale et la maintenir encore dans cette position, alors que l'éjecteur (29) continue à expulser la greffe (21),
e) pour stopper l'éjecteur (29) dans une position frontale et le maintenir encore dans cette position, alors que la canule (1) est rentrée,
f) pour faire reculer l'éjecteur (29) et la canule (1) jusqu'à ce que leurs positions arrière respectives aient été atteintes,
où le récipient (20) est fixé dans sa position de retrait, alors que l'éjecteur (29) expulse la greffe (21) du compartiment du récipient (20), et l'éjecteur (29) est ramené en arrière à sa position initiale.

2. Appareil selon la revendication 1, **caractérisé en ce que** les moyens de commande contrôlent les dispositifs, de manière telle qu'entre les étapes b) et c), la canule (1) soit déplacée jusqu'à une première position et maintenue dans cette position pendant un intervalle de temps qui est préprogrammé ou sélectionné en cours d'utilisation.

3. Appareil selon la revendication 1 ou 2, **caractérisé en ce que** les moyens de commande contrôlent les dispositifs, de manière telle qu'entre les étapes c) et d), la canule (1) soit reculée sur une distance prédéterminée.

4. Appareil selon l'une quelconque des revendications précédentes, **caractérisé en ce que** les dispositifs de transport pour la canule (1), l'éjecteur (29) et le récipient (20) sont entraînés par des servomoteurs ou par des moteurs pas à pas, et les moyens de commande sont composés d'une commande logique programmée ou d'une commande par microprocesseur.

5. Appareil selon l'une quelconque des revendications précédentes, **caractérisé en ce que** les moyens de commande reçoivent des signaux de position et de vitesse provenant des émetteurs de signaux qui accusent réception du fait que chaque mouvement des dispositifs a été effectué et que les dispositifs ont atteint leurs positions frontales et/ou arrière et/ou leurs positions intermédiaires.

6. Appareil selon l'une quelconque des revendications précédentes, **caractérisé en ce que** les moyens de commande comprennent des programmes permettant de commander des interruptions de production et/ou de réenrouler le récipient (20) et/ou de réparer et de tester l'appareil.
